# EUROPÄISCHE PATENTSCHRIFT

(11) **EP 2 049 037 B1**
(45) Veröffentlichungstag und Bekanntmachung des Hinweises auf die Patenterteilung: **17.03.2010**
(21) Anmeldenummer: 06777411.7
(22) Anmeldetag: 21.06.2006
(51) Int. Cl.: A61B 19/08, A61B 1/00, A61B 19/00

(54) **VORRICHTUNG ZUM EINFÜHREN UND POSITIONIEREN VON CHIRURGISCHEN INSTRUMENTEN**
DEVICE FOR INTRODUCING AND POSITIONING SURGICAL INSTRUMENTS
DISPOSITIF POUR INTRODUIRE ET POSITIONNER DES INSTRUMENTS CHIRURGICAUX

(43) Veröffentlichungstag der Anmeldung: 22.04.2009
(73) Patentinhaber: Steffen, Rudolf, 3011 Bern (CH)
(72) Erfinder: Steffen, Rudolf, 3011 Bern (CH)
(74) Vertreter: Scheuzger, Beat Otto
(86) Internationale Anmeldenummer: PCT/EP2006/063425
(87) Internationale Veröffentlichungsnummer: WO 2007/147439

(56) Entgegenhaltungen:
- WO-A-02/00121
- US-A1- 2003 028 178
- US-A1- 2005 080 430

## Beschreibung

### Technisches Gebiet

Die vorliegende Erfindung bezieht sich auf eine Vorrichtung zum Einführen und Positionieren von chirurgischen Instrumenten. Insbesondere bezieht sich die vorliegende Erfindung auf eine Vorrichtung zum Einführen von chirurgischen Instrumenten in den Körper eines Patienten durch eine geeignete Körperöffnung und deren Positionieren an der Einsatzstelle.

### Stand der Technik

Operative Eingriffe in den menschlichen oder tierischen Körper sind eines der Mittel der modernen Medizin, welche eingesetzt werden, um eine raschere Genesung des Patienten zu ermöglichen. Diese Massnahmen ermöglichen oft eine vollständige Heilung der Krankheit, verursachen aber auch selber ein relativ grosses Trauma im betroffenen Gewebe, von welchem sich der Körper des Patienten anschliessend erholen muss. Dabei können viele postoperative Beschwerden auf die Schnitte in der Haut und anderen Weichteilen des Körpers zurückgeführt werden. Aus diesem Grund war es schon immer ein Ziel der chirurgischen Behandlung, möglichst geringe Beschwerden nach der Operation auszulösen, d.h. den chirurgischen Eingriff möglichst schonend zu gestalten.

Deshalb wurde vor einiger Zeit die so genannte laparoskopische Chirurgie eingeführt. Bei dieser Operationsmethode werden bei minimalen Schnitten und mit Hilfe eines optischen Instruments Eingriffe innerhalb der Bauchhöhle vorgenommen. Diese Methode wurde zunächst zur chirurgischen Entfernung der Gallenblase, später auch zur Durchführung komplexerer Operationen eingesetzt. Die klaren Vorteile dieser Operationstechnik im Bezug auf die Erholung des Patienten nach dem Eingriff führten zur Entwicklung so genannter minimalinvasiver Operationsverfahren auch auf anderen Gebieten, so dass diese minimalinvasiven Operationstechniken heute viele konventionelle Operationsverfahren (mit ausgedehnterem Schnitt) bereits verdrängt haben. So werden heute oft auch thorakoskopische oder knochenstabilisierende Operationen auf diese Art und Weise durchgeführt, und auch bei Schilddrüsenoperationen bestehen Tendenzen zu minimalinvasiven Zugängen, obwohl sie sich in der Breite noch nicht durchgesetzt haben.

Kleinere Schnitte und geringere Verletzungen der Weichteile beim Zugang führen meist zu geringeren postoperativen Schmerzen und meistens auch zu einer rascheren vollständigen Genesung des Patienten. Ausserdem bieten laparoskopische oder thorakoskopische Operationsverfahren die Möglichkeit, eine detaillierte Untersuchung des Bauch- oder Brustraumes zu Diagnosezwecken vorzunehmen, was bei einem konventionellen Zugang normalerweise nicht möglich wäre. Zudem wird manchmal eine laparoskopische Untersuchung beispielsweise auch vor einer komplexeren Operation durchgeführt, um eine Statuserhebung durchzuführen und somit das weitere Vorgehen besser planen zu können.

Bei den minimalinvasiven Operationsverfahren werden diverse chirurgische Instrumente verwendet, die eigens für diesen Einsatz entwickelt wurden. In der Gruppe solcher chirurgischen Instrumente spielen die so genannten Klammernahtgeräte oder Stapler eine grosse Rolle. Diese Klammernahtgeräte sind komplexe medizinische Vorrichtungen, in denen verschiedene Funktionalitäten an einer Stelle vereint sind. So können mittels eines Klammernahtgeräts Teile kranker oder beschädigter Organe entfernt werden (Resektion), Schnitte in Organen und Geweben gemacht werden (Transektion), oder jedoch Verbindungen zwischen Blutgefässen, Nerven und Hohlorganen hergestellt werden (Anastomose). Der Vorteil dieser Geräte ist insbesondere eine schnellere und effizientere Operation, da verschiedene Operationsphasen mit einem einzigen Instrument durchgeführt werden können.

Jedoch weisen insbesondere die herkömmlichen Klammernahtgeräte meistens den Nachteil auf, dass sie wegen ihrer Komplexität oft relativ gross gebaut sind. Zudem hat der Vorderteil des Klammernahtgeräts mit dem eigentlichen Arbeitseinsatz, aber auch Vorderteile anderer chirurgischen Instrumente, oft eine sehr unregelmässige Form, so dass sich das Einführen dieser chirurgischen Instrumente in den Körper des Patienten, sowie deren genaues Positionieren am Einsatzort manchmal als sehr schwierig gestalten. Darüber hinaus besteht beispielsweise beim Vordringen des Klammernahtgeräts die Gefahr, dass das umliegende Gewebe durch den Arbeitseinsatz verletzt wird, was die Genesungszeit des Patienten zum Teil erheblich verlängern könnte.

Eine Lösung dieses Problems wird in WO 02/00121 vorgeschlagen. Da ist von einem Gleitschutz für den Gehäusekopf medizinischer Instrumente die Rede, welcher eine am Gehäusekopf überkragend befestigte Abschlussvorrichtung aufweist Diese Abschlussvorrichtung kann fernbetätigbar in wenigstens zwei Abschnitten vom Gehäusekopf entfernt werden. Diese Abschlussvorrichtung kann ausserdem insbesondere aus einem elastisch verformbaren Material hergestellt, und die wenigstens zwei Abschnitte symmetrisch ausgebildet werden. Allerdings werden die wenigstens zwei Abschnitte getrennt aus dem Operationsbereich herausgezogen, so dass auch bei der Verwendung des vorgeschlagenen Gleitschutz eine akute Verletzungsgefahr durch scharfe Bruchkanten besteht.

Eine andere Vorrichtung wird in US 2003/0028178 dargestellt. Diese Vorrichtung besteht aus einer flexiblen Hülse, welche über das endoskopische Instrument übergezogen und durch mindestens ein elastisches Band am distalen Ende des Instrumentes befestigt. Zur Befreiung des endoskopischen Instruments wird dieses mindestens eine Band durchtrennt (beispielsweise mit einem anderen chirurgischen Instrument), die Hülse entlang Perforationen abgerissen und aus dem Operationsbereich entfernt. Auch diese Vorrichtung löst aber das Problem nicht, dass bei deren Ausziehen aus dem Operationsbereich mögliche Verletzungen entstehen können.

### Offenbarung der Erfindung

Es ist daher eine Aufgabe der vorliegenden Erfindung, eine neue Vorrichtung zum Einführen und Positionieren von chirurgischen Instrumenten vorzuschlagen, welche nicht die Nachteile des Standes der Technik aufweisen. Es ist insbesondere eine Aufgabe der vorliegenden Erfindung, eine Vorrichtung bereitzustellen, welche ein präzises, einfaches und schnelles Einführen von chirurgischen Instrumenten, beispielsweise von einem Klammernahtgerät, und deren anschliessendes präzises Positionieren im Körper des Patienten zu ermöglichen, ohne jedoch eine zusätzliche Verletzungsgefahr zu kreieren.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Insbesondere werden die Ziele der Erfindung dadurch erreicht, dass bei einer Vorrichtung zum Einführen und Positionieren von chirurgischen Instrumenten in den Körper eines Patienten, mit einer Aussenumhüllung, in welche Aussenumhüllung mindestens ein Vorderteil des chirurgischen Instruments ensetzbar ist, und welche Aussenumhüllung zum Positionieren des chirurgischen Instruments an der Einsatzstelle entfernbar ist, mittels einer Zuganordnung und/oder Perforationen an der Spitze der Aussenumhüllung eine Öffnung erzeugbar ist, und die Aussenumhüllung mittels der Zuganordnung in einem Stück über den Vorderteil des chirurgischen Instruments nach hinten herüberziehbar ist.

Eine solche Vorrichtung hat insbesondere den Vorteil, dass mindestens der Vorderteil des chirurgischen Instruments während dessen Einführens in den Körper des Patienten durch die Aussenumhüllung geschützt ist. Da der Vorderteil von vielen chirurgischen Instrumenten, wie zum Beispiel von einem Klammernahtgerät, oft eine unregelmässige Form aufweist, ist dessen Einführen in den Körper des Patienten und das präzise Positionieren am Einsatzort manchmal äusserst schwierig. Ausserdem können durch das Vordringen des chirurgischen Instruments Schäden am umliegenden Gewebe entstehen, welche zu Komplikationen und einer längeren postoperativen Erholung des Patienten führen können. Die Aussenumhüllung löst dieses Problem dadurch, dass sie den vorderen Teil des einzuführenden bzw. des zu positionierenden chirurgischen Instruments vollständig oder teilweise bedeckt und so ein erleichtertes Einführen ermöglicht. Selbstverständlich muss aber diese Aussenumhüllung entfernt werden, um den Einsatz des chirurgischen Instruments zu ermöglichen, so dass sie mittels einer geeigneten Zuganordnung vom Vorderteil des chirurgischen Instruments weggezogen werden kann. Dabei wird aber diese Aussenumhüllung in einem einzigen Stück über den Vorderteil des Instruments nach hinten weggezogen. Dadurch kann der Vorderteil des chirurgischen Instruments auf einfachste Weise von dieser Aussenumhüllung befreit werden. Auf der anderen Seite kann die Aussenumhüllung beim Wegziehen dem Verlauf des chirurgischen Instruments folgen, womit weitere potentielle Verletzungen des umliegenden Gewebes durch Bruchkanten an der Perforationsbruchstellen verhindert werden können.

In einer anderen Ausführungsvariante ist die Aussenumhüllung mittels einer Rückführanordnung in die Ausgangsposition rückführbar. Diese Ausführungsvariante hat insbesondere den Vorteil, dass beispielsweise nach der Beendigung des chirurgischen Eingriffs der Vorderteil des chirurgischen Instruments wieder mittels der Aussenumhüllung bedeckt werden kann, bevor das chirurgische Instrument aus dem Körper des Patienten herausgenommen wird. Dadurch kann das chirurgische Instrument nicht nur wesentlich einfacher aus dem Körper des Patienten herausgeführt werden, sondern es können auch weitere Verletzungen des umliegenden Gewebes verhindert werden.

In einer weiteren Ausführungsvariante ist die Aussenumhüllung automatisch in die Ausgangsposition rückführbar. Diese Ausführungsvariante hat insbesondere den Vorteil, dass die Rückführung der Aussenumhüllung in die Ausgangsposition automatisch geschehen kann, und dass sie nicht durch den Chirurgen vorgenommen werden muss. So kann der operative Eingriff mit dem freigestellten chirurgischen Instrument vorgenommen werden, während die Aussenumhüllung anschliessend automatisch wieder über den Vorderteil des chirurgischen Instrumenten hinübergezogen wird. Das chirurgische Instrument kann danach besonders einfach wieder aus dem Körper des Patienten entfernt werden.

In einer anderen Ausführungsvariante ist die Aussenumhüllung mittels einer Arretieranordnung an einem Schaft des chirurgischen Instruments befestigbar. Diese Ausführungsvariante hat insbesondere den Vorteil, dass die Aussenumhüllung nach dem Einführen des chirurgischen Instruments in den Körper des Patienten und nach dessen Zurückziehen am Einsatzort in einer fixen Position befestigt werden kann. Dadurch kann die Aussenumhüllung insbesondere während des operativen Eingriffs an einer fixen Position gehalten werden, so dass die Operation keinesfalls beeinträchtigt wird. Des Weiteren kann die Aussenumhüllung gemäss dieser Ausführungsvariante der vorliegenden Erfindung erst nach dem operativen Eingriff zusammen mit dem chirurgischen Instrument aus dem Körper des Patienten herausgezogen werden. Dadurch kann auch Zeit gespart werden, wodurch die Chancen für eine schnelle Genesung des Patienten noch weiter gesteigert werden können.

In einer wieder anderen Ausführungsvariante ist der Vorderteil der Aussenumhüllung geschlossen. Diese Ausführungsvariante hat insbesondere den Vorteil, dass der Vorderteil des chirurgischen Instruments auf eine besonders vorteilhafte Art und Weise vollständig geschützt werden kann, und beispielsweise erst nach dem Einführen des chirurgischen Instrumenten in den Körper des Patienten von der Aussenumhüllung befreit werden kann. Ausserdem kann die Bewegung der Aussenumhüllung der Vorrichtung zum Einführen und Positionieren von chirurgischen Instrumenten auf eine besonders einfache Art und Weise ermöglicht werden.

In einer weiteren Ausführungsvariante ist die Aussenumhüllung aus einem Edelmetall und/oder aus Kunststoff verschiedenen Härtegrades gebildet. Diese Ausführungsvariante hat insbesondere den Vorteil, dass sowohl Edelmetall als auch Kunststoff bereits bestens bekannte und geprüfte Materialien zur Herstellung von chirurgischen Instrumenten sind. Ausserdem besitzen beide dieser Materialien besonders vorteilhafte Eigenschaften, welche insbesondere beim Einsatz der Vorrichtung zum Einführen und Positionieren von chirurgischen Instrumenten in den Körper des Patienten zum Ausdruck kommen und auch die Zulassungsvorschriften für chirurgische Instrumente vollkommen erfüllen.

In einer anderen Ausführungsvariante ist die Aussenumhüllung rotationssymmetrisch. Diese Ausführungsvariante hat insbesondere den Vorteil, dass die Position der Vorrichtung zum Einführen und Positionieren von chirurgischen Instrumenten in den Körper des Patienten gemäss dieser Ausführungsvariante der vorliegenden Erfindung relativ zum chirurgischen Instrument selbst keine Rolle spielt. Ausserdem kann beim Einführen auch die Vorstossrichtung und/oder der -winkel im Körper des Patienten geändert werden, ohne dass das Einführen dadurch beeinträchtigt oder gar verunmöglicht wäre. Darüber hinaus kann eine rotationssymmetrische Form der Aussenumhüllung besonders vorteilhaft hergestellt werden, wodurch die Gesamtkosten der Vorrichtung zum Einführen und Positionieren von chirurgischen Instrumenten in den Körper des Patienten gemäss dieser Ausführungsvariante der vorliegenden Erfindung reduziert werden können.

In einer wieder anderen Ausführungsvariante weist die Aussenumhüllung im Wesentlichen eine olivenförmige und/oder eine konische Form auf. Diese Ausführungsvariante hat insbesondere den Vorteil, dass sich diese Form besonders vorteilhaft zum Einführen in den Körper eines Patienten eignen. Das chirurgische Instrument kann ohne grossen Widerstand zum Einsatzort geführt werden. Diese besonderen Formen ermöglichen ausserdem ein Einführen mit möglichst kleinen Beeinträchtigungen des umliegenden Gewebes, welches durch den sich von der Spitze aus vergrössemden Umfang der Aussenumhüllung gemäss dieser Ausführungsvariante beim Vorstossen auf die Seite geschoben wird.

In einer weiteren Ausführungsvariante ist die Aussenumhüllung mittels der Zuganordnung vom Handgriff und/oder vom Bedienungsgriff des chirurgischen Instruments kontrollierbar. Diese Ausführungsvariante hat insbesondere den Vorteil, dass Operationen unter Einsatz der Vorrichtung zum Einführen und Positionieren von chirurgischen Instrumenten in den Körper des Patienten gemäss dieser Ausführungsvariante der vorliegenden Erfindung auf die herkömmliche Art ausgeführt werden können. Die praktizierenden Ärzte müssen nicht neu für den Umgang mit der erfindungsgemässen Vorrichtung geschult werden und müssen zudem ihre gewohnten Operationspraxis nicht ändern. Ausserdem kann die Aussenumhüllung gemäss dieser Ausführungsvariante nötigenfalls auch noch während der Operation kontrolliert und gesteuert werden.

An dieser Stelle soll festgehalten werden, dass sich die vorliegende Erfindung neben der erfindungsgemässen Vorrichtung auch auf ein entsprechendes Verfahren zum Einführen und Positionieren von chirurgischen Instrumenten in den Körper eines Patienten bezieht.

### Kurze Beschreibung der Zeichnungen

Nachfolgend werden die Ausführungsvarianten der vorliegenden Erfindung anhand von Beispielen beschrieben. Die Beispiele der Ausführungen werden durch folgende beigelegte Figuren illustriert:
Figur 1 zeigt eine perspektivische schematische Darstellung eines chirurgischen Instruments aus dem Stand der Technik.
Figur 2 zeigt eine perspektivische schematische Darstellung einer Vorrichtung zum Einführen und Positionieren von chirurgischen Instrumenten in den Körper eines Patienten gemäss einer Ausführungsvariante der vorliegenden Erfindung beim Einführen des chirurgischen Instruments in den Körper des Patienten.
Figuren 3A und 3B zeigen schematisch einen Querschnitt über zwei Vorrichtungen zum Einführen und Positionieren von chirurgischen Instrumenten in den Körper eines Patienten gemäss zwei Ausführungsvarianten der vorliegenden Erfindung.
Figur 4 zeigt eine perspektivische schematische Darstellung einer Vorrichtung zum Einführen und Positionieren von chirurgischen Instrumenten in den Körper eines Patienten gemäss einer Ausführungsform der vorliegenden Erfindung während des operativen Eingriffs.

### Ausführungsformen der Erfindung

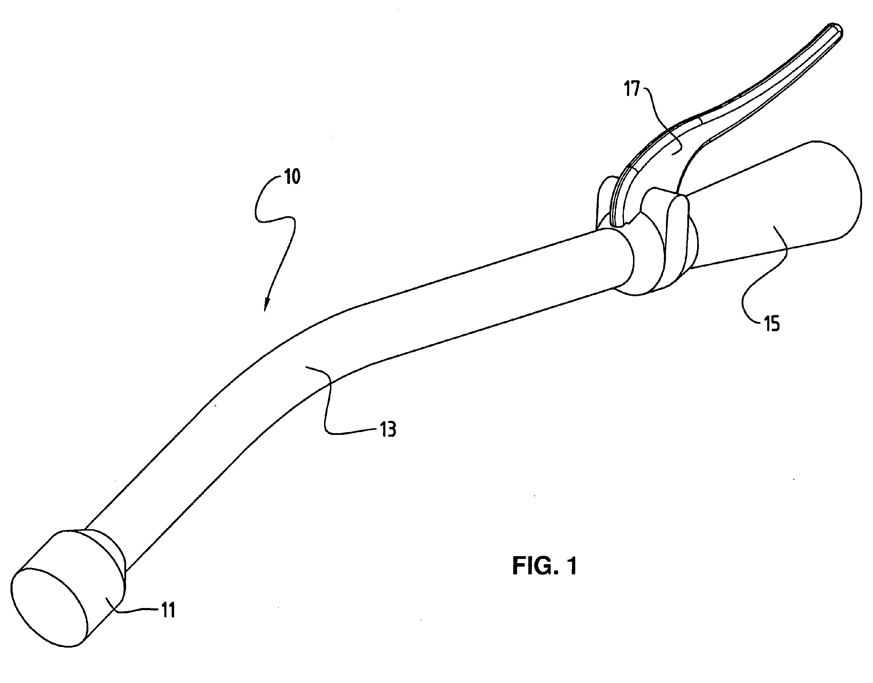

Figur 1 zeigt ein chirurgisches Instrument, genauer ein Klammernahtgerät, aus dem Stand der Technik. In Figur 1 bezieht sich das Bezugszeichen 10 auf das chirurgische Instrument selbst, das Bezugszeichen 11 auf den Vorderteil des chirurgischen Instruments 10 und das Bezugszeichen 13 auf den Schaft des chirurgischen Instruments 10. Der Vorderteil 11 des chirurgischen Instruments 10 kann insbesondere verschiedene Arbeitseinsätze umfassen, welche verschiedene Funktionen ausführen können, und welche daher auch verschiedene Formen und/oder Grössen aufweisen können. Auch können diese Arbeitseinsätze aus verschiedensten Materialien hergestellt werden, welche gleich oder anders sein können als die Materialien des chirurgischen Instruments 10 selbst. Diese Arbeitseinsätze können insbesondere nach Bedarf auch ausgetauscht werden, so dass das chirurgische Instrument 10 für verschiedene Aufgaben verwendet werden kann. Ausserdem bezieht sich das Bezugszeichen 15 in Figur 1 auf den Handgriff des chirurgischen Instruments 10 und das Bezugszeichen 17 auf den Bedienungsgriff des chirurgischen Instruments 10, welche dem praktizierenden Arzt zum Halten und Kontrollieren des chirurgischen Instruments 10 bzw. des entsprechenden Arbeitseinsatzes am Vorderteil 11 des chirurgischen Instruments 10 während des operativen Eingriffs dienen können.

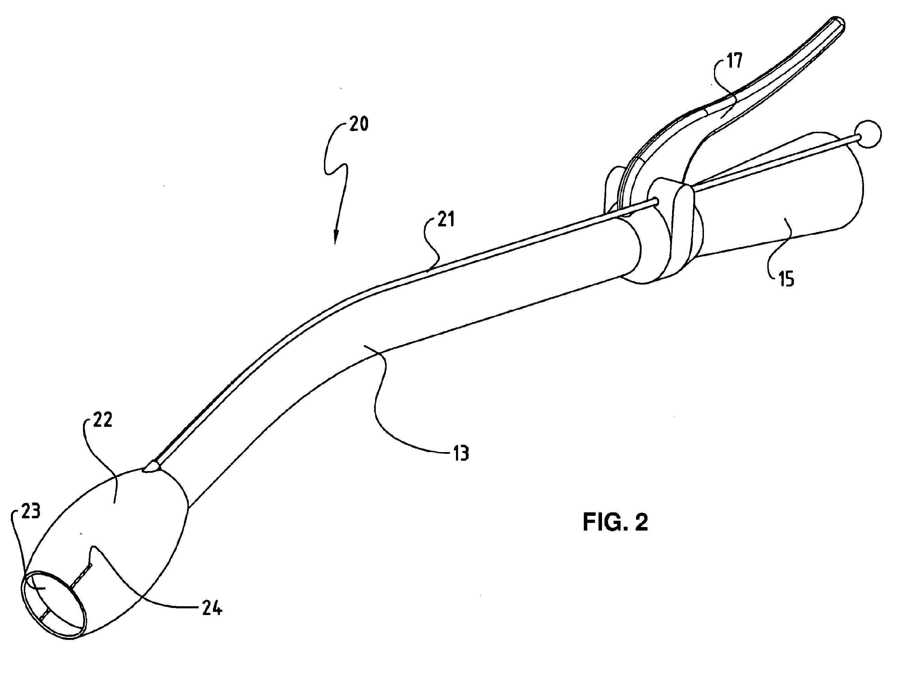

In Figur 2 wird eine Vorrichtung 20 zum Einführen und Positionieren von chirurgischen Instrumenten 10 in den Körper eines Patienten gemäss einer Ausführungsvariante der vorliegenden Erfindung beim Einführen des chirurgischen Instruments 10 in den Körper des Patienten illustriert. In Figur 2 beziehen sich die Bezugszeichen 13, 15 und 17 wie vorangehend in Figur 1 auf den Schaft, den Handgriff und den Bedienungsgriff des chirurgischen Instruments 10. Darüber hinaus bezieht sich das Bezugszeichen 21 auf eine Zuganordnung und das Bezugszeichen 22 auf eine Aussenumhüllung. Die Aussenumhüllung 22 umschliesst den Innenraum 23, in welchem zumindest der Vorderteil 11 des chirurgischen Instruments 10 mit dem dazugehörenden Arbeitseinsatz untergebracht werden kann. Schliesslich bezieht sich das Bezugszeichen 24 auf die Perforation an der Spitze der Aussenumhüllung 22 des chirurgischen Instruments 10. Die Zuganordnung 21 ist mit der Aussenumhüllung 22 verbunden, so dass die Aussenumhüllung 22 mittels der Zuganordnung 21 nach hinten gezogen werden kann. Die Zuganordnung 21 kann beispielsweise als ein dünner Strang ausgebildet sein, aus gleichem Material wie die Aussenumhüllung 22. Selbstverständlich kann aber die Zuganordnung 21 auch eine andere Form haben oder aus einem anderen Material hergestellt sein. Insbesondere kann die Zuganordnung 21 mit dem Handgriff 15 und/oder mit dem Bedienungsgriff des chirurgischen Instruments 10 verbunden werden, so dass die Aussenumhüllung 22 vom Handgriff 15 des chirurgischen Instruments 10 aus kontrolliert werden kann.

Die Aussenumhüllung 22 kann beispielsweise eine olivenförmige Form aufweisen, aber auch in einer konischen oder anderen Form ausgebildet sein. Insbesondere kann die Aussenumhüllung 22 auch rotationssymmetrisch sein, beispielsweise um ihre Längsachse. Diese besondere Form der Aussenumhüllung 22 ermöglicht ein besonders einfaches Einführen des chirurgischen Instruments 10 in den Körper eines Patienten, aber auch eine einfachere und damit günstigere Herstellung. Allerdings möchten wir an dieser Stelle betonen, dass sowohl die Aussenumhüllung 22 als auch die Zuganordnung 21 oder beliebige andere Bestandteile der erfindungsgemässen Vorrichtung zum Einführen und Positionieren von chirurgischen Instrumenten 10 in den Körper eines Patienten selbstverständlich auch ganz andere Formen bzw. Funktionsweisen aufweisen können. So kann beispielsweise insbesondere die Aussenumhüllung 22 am vorderen Ende beim Einführen des chirurgischen Instruments 10 auch vollständig geschlossen sein. Auch kann die Zuganordnung 21 nicht nur an einer einzigen, sondern gleichzeitig an mehreren Stellen mit der Aussenumhüllung 22 verbunden sein.

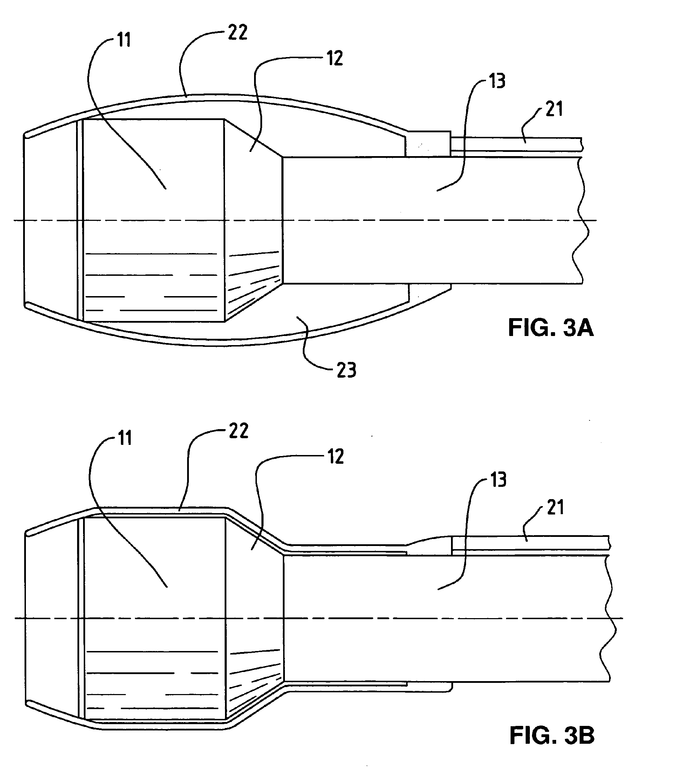

Figuren 3A und 3B zeigen zwei besondere Ausführungsvarianten des vordersten Teils der Vorrichtung 20 zum Einführen und Positionieren von chirurgischen Instrumenten 10 in den Körper eines Patienten. Auch bei diesen Figuren bezieht sich das Bezugszeichen 11 auf den Vorderteil des chirurgischen Instruments 10, das Bezugszeichen 12 auf die hintere Seite des Vorderteils des chirurgischen Instruments 10 und das Bezugszeichen 13 auf den Schaft des chirurgischen Instruments 10. Des Weiteren bezieht sich das Bezugszeichen 21 auf die Zuganordnung, welche mit der Aussenumhüllung 22 der Vorrichtung zum Einführen und Positionieren von chirurgischen Instrumenten 10 verbunden ist. In Figur 3A bezieht sich das Bezugszeichen 23 auf den Innenraum, welcher von der Aussenumhüllung 22 gebildet wird. Die Aussenumhüllung 22 kann jeweils insbesondere den Vorderteil 11 des chirurgischen Instruments 10 und einen Teil dessen Schaftes 13 umhüllen. Die Aussenumhüllung 22 kann dabei insbesondere eine Form aufweisen, bei welcher ihr Querschnitt an der Spitze kleiner ist als der Querschnitt an einer Stelle, welche dem Schaft 13 des chirurgischen Instruments 10 näher steht. Dadurch kann die Aussenumhüllung 22 beim Vordringen im Körper des Patienten zum Einsatzort auf einen möglichst kleinen Widerstand stossen. Wie in Figur 3A dargestellt kann die Aussenumhüllung 22 grösser sein als der Vorderteil 11 des chirurgischen Instruments 10 mit dem dazugehörenden Arbeitseinsatz, so dass zwischen der Aussenumhüllung 22 und dem Vorderteil 11 des chirurgischen Instruments 10 ein Zwischenraum 23 entsteht. Andererseits kann, wie in Figur 3B, die Aussenumhüllung 22 an die Form des Vorderteils 11 des chirurgischen Instruments 10 aufs Genauste angepasst sein. Die Aussenumhüllung 22 kann an der Spitze, wie dargestellt, eine Öffnung aufweisen, aber auch vollständig geschlossen sein.

Beim Einführen des chirurgischen Instruments 10 in den Körper des Patienten dient die Aussenumhüllung 22 als eine Art Schild, welcher einerseits den empfindlichen Vorderteil 11 des chirurgischen Instruments 10 mit dem dazugehörenden Arbeitseinsatz vor Beschädigung schützt, und andererseits dank ihrer besonderen Form das Einführen des chirurgischen Instruments 10 in den Körper des Patienten auf eine positive Weise unterstützt. Ausserdem schützt die besondere Form der Aussenumhüllung 22 auch das umliegende Gewebe vor Verletzungen durch das chirurgische Instrument 10. Nachdem das chirurgische Instrument 10 mit der Aussenumhüllung 22 an den Einsatzort im Körper des Patienten geführt worden ist, kann die Zuganordnung 21 betätigt werden, so dass der Zug auf die mit der Zuganordnung 21 verbundene Aussenumhüllung 22 übertragen wird. Die Aussenumhüllung 22 kann beispielsweise an der Spitze eine oder mehrere Perforationen 24 aufweisen, welche durch die Zugwirkung der Zuganordnung 21 reissen und die Bewegungen der Aussenumhüllung 22 ermöglichen. Es sind aber selbstverständlich auch andere Ausführungsvarianten möglich und denkbar. Mittels der Zuganordnung 21 kann also der Vorderteil 11 des chirurgischen Instruments 10 von der Aussenumhüllung 22 befreit werden. Anschliessend kann das chirurgische Instrument 10 wie gewohnt verwendet werden. Die Aussenumhüllung 22 kann nach dem Wegziehen vom Vorderteil 11 des chirurgischen Instruments 10 beispielsweise auf dem gleichen Wege aus dem Körper des Patienten entfernt werden, oder aber während des operativen Eingriffs in der Nähe des chirurgischen Instruments 10 platziert werden und erst zusammen mit dem chirurgischen Instrument 10 selbst aus dem Körper des Patienten herausgenommen werden.

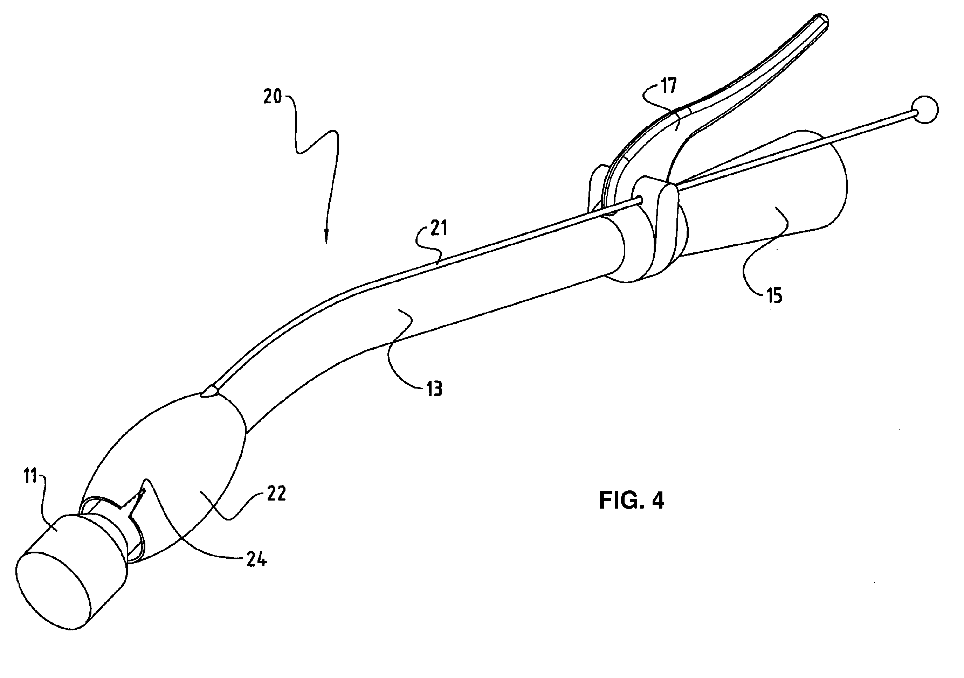

Vorteilhaft kann die Aussenumhüllung 22 aber nach der Freigabe des Vorderteils 11 des chirurgischen Instruments 10 nach hinten, d.h. über den Schaft 13 des chirurgischen Instruments 10 gezogen werden. Eine Vorrichtung 20 zum Einführen und Positionieren von chirurgischen Instrumenten 10 in den Körper eines Patienten gemäss dieser Ausführungsvariante der vorliegenden Erfindung ist in Figur 4 dargestellt. In Figur 4 bezieht sich das Bezugszeichen 11 erneut auf den Vorderteil des chirurgischen Instruments 10, das Bezugszeichen 13 auf den Schaft des chirurgischen Instruments 10, das Bezugszeichen 15 auf den Handgriff des chirurgischen Instruments 10 und das Bezugszeichen 13 auf den Bedienungsgriff des chirurgischen Instruments 10. Das Bezugszeichen 21 bezieht sich erneut auf die Zuganordnung, welche mit der Aussenumhüllung 22 der Vorrichtung zum Einführen und Positionieren von chirurgischen Instrumenten 10 verbunden ist. Das Bezugszeichen 24 bezieht sich schliesslich auf die Perforation 24 an der Spitze der Aussenumhüllung 22.

Die Vorrichtung 20 zum Einführen und Positionieren von chirurgischen Instrumenten 10 in den Körper eines Patienten kann insbesondere auch weitere Bestandteile umfassen, welche in den beiliegenden Zeichnungen nicht dargestellt sind. So kann die Aussenumhüllung 22 beispielsweise mittels einer Rückführanordnung in die Ausgangsposition zurückgeführt werden. Diese Rückführanordnung kann beispielsweise ähnlich wie die Zuganordnung 21 als ein Strang aus einem geeigneten Material ausgebildet sein, aber auch als eine eigenständige komplexere Anordnung. Auch kann die Vorrichtung 20 zum Einführen und Positionieren von chirurgischen Instrumenten 10 in den Körper eines Patienten zum Beispiel eine Anordnung umfassen, welche zur automatischen Zurückführung der Aussenumhüllung 22 in die Ausgangsposition verwendet werden kann. Dabei kann diese Anordnung insbesondere als eine mechanische Feder ausgebildet sein, oder als jede andere geeignete Anordnung. Schliesslich kann die Aussenumhüllung 22 mittels einer Arretieranordnung beispielsweise am Schaft 13 des chirurgischen Instruments 10 befestigt werden, so dass sie während des operativen Eingriffs nicht verrutschen und Schäden anrichten kann.

Zum Schluss sei darauf hingewiesen, dass die hier beispielhaft beschriebenen Ausführungsvarianten nur eine Auswahl an möglichen Realisierungen der erfindungsgemässen Gedanken darstellen und keinesfalls als limitierend angeschaut werden sollen. Der Fachmann wird verstehen, dass viele andere Implementierungen der Erfindung möglich sind, ohne dass die Merkmale der Erfindung vernachlässigt werden.

## Patentansprüche

1. Vorrichtung (20) zum Einführen und Positionieren von chirurgischen Instrumenten (10) in den Körper eines Patienten, mit einer Aussenumhüllung (22) in welche Aussenumhüllung (22) mindestens ein Vorderteil (11) des chirurgischen Instruments (10) einsetzbar ist, und welche Aussenumhüllung (22) zum Positionieren des chirurgischen Instruments (10) an der Einsatzstelle entfernbar ist, **dadurch gekennzeichnet,**
**dass** mittels einer Zuganordnung (21) und Perforationen (24) an der Spitze der Aussenumhüllung (22) eine Öffnung erzeugbar ist, und
**dass** die Aussenumhüllung (22) mittels der Zuganordnung (21) und reissen der Perforationen (24) in einem Stück über den Vorderteil (11) des chirurgischen Instruments (10) nach hinten herüberziehbar ist.

2. Vorrichtung (20) nach Anspruch 1, **dadurch gekennzeichnet, dass** die Aussenumhüllung (22) mittels einer Rückführanordnung in die Ausgangsposition rückführbar ist.

3. Vorrichtung (20) nach Anspruch 2, **dadurch gekennzeichnet, dass** die Aussenumhüllung (22) automatisch in die Ausgangsposition rückführbar ist.

4. Vorrichtung (20) nach einem der Ansprüche 1 bis 3, **dadurch gekennzeichnet, dass** die Aussenumhüllung (22) mittels einer Arretieranordnung an einem Schaft (13) des chirurgischen Instruments (10) befestigbar ist.

5. Vorrichtung (20) nach einem der Ansprüche 1 bis 4, **dadurch gekennzeichnet, dass** der Vorderteil der Aussenumhüllung (22) geschlossen ist.

6. Vorrichtung (20) nach einem der Ansprüche 1 bis 5, **dadurch gekennzeichnet, dass** die Aussenumhüllung (22) aus einem Edelmetall und/oder aus Kunststoff verschiedenen Härtegrade gebildet ist.

7. Vorrichtung (20) nach einem der Ansprüche 1 bis 8, **dadurch gekennzeichnet, dass** die Aussenumhüllung (22) rotationssymmetrisch ist.

8. Vorrichtung (20) nach einem der Ansprüche 1 bis 7, **dadurch gekennzeichnet, dass** die Aussenumhüllung (22) im Wesentlichen eine olivenförmige und/oder eine konische Form aufweist.

9. Vorrichtung (20) nach einem der Ansprüche 1 bis 8, **dadurch gekennzeichnet, dass** die Aussenumhüllung (22) mittels der Zuganordnung (21) vom Handgriff (15) und/oder vom Bedienungsgriff (17) des chirurgischen Instruments (10) kontrollierbar ist.

## Claims

1. Device (20) for inserting and positioning surgical instruments (10) in the body of a patient, with an outer cover (22), into which outer cover (22) at least a front part (11) of the surgical instrument (10) is insertable, and which outer cover (22) is removable for positioning the surgical instrument (10) at the point of application, **characterised in that**
an opening is able to be created at the tip of the outer cover (22) by means of a pulling device (21) and perforations (24), and
the outer cover (22) is able to be pulled backwards, in one piece, over the front part (11) of the surgical instrument (10) by means of the pulling device (21) and tearing of the perforations (24).

2. Device (20) according to claim 1, **characterised in that** the outer cover (22) is able to be returned to the starting position by means of a return device.

3. Device (20) according to claim 2, **characterised in that** the outer cover (22) is able to be returned automatically to the starting position.

4. Device (20) according to one of the claims 1 to 3, **characterised in that** the outer cover (22) is attachable to a shaft (13) of the surgical instrument (10) by means of a locking device.

5. Device (20) according to one of the claims 1 to 4, **characterised in that** the front part of the outer cover (22) is closed.

6. Device (20) according to one of the claims 1 to 5, **characterised in that** the outer cover (22) is made of a noble metal and/or plastic of different degrees of hardness.

7. Device (20) according to one of the claims 1 to 8 <sic. 6>, **characterised in that** the outer cover (22) is rotationally symmetrical.

8. Device (20) according to one of the claims 1 to 7, **characterised in that** the outer cover (22) has substantially an olive-shaped and/or conical form.

9. Device (20) according to one of the claims 1 to 8, **characterised in that** the outer cover (22) is controllable by means of the pulling device (21), using the handle (15) and/or the operating handle (17) of the surgical instrument (10).

## Revendications

1. Dispositif (20) pour introduire et positionner des instruments chirurgicaux (10) dans le corps d'un patient avec une enveloppe externe (22), enveloppe externe (22) dans laquelle au moins une partie avant (11) de l'instrument chirurgical (10) peut être introduite et enveloppe externe (22) pour le positionnement de l'instrument chirurgical (10) qui peut être retirée au point d'introduction, **caractérisé en ce**
**qu'**au moyen d'un dispositif de traction (21) et des perforations (24), une ouverture peut être réalisée à la pointe de l'enveloppe externe (22) et
en ce que l'enveloppe externe (22) peut être tirée d'une seule pièce vers l'arrière au moyen du dispositif de traction (21) et par arrachage des perforations (24) sur la partie avant (11) de l'instrument chirurgical (10).

2. Dispositif (20) selon la revendication 1, **caractérisé en ce que** l'enveloppe externe (22) peut être ramenée en position initiale au moyen d'un dispositif de retour.

3. Dispositif (20) selon la revendication 2, **caractérisé en ce que** l'enveloppe externe (22) peut être ramenée automatiquement à la position initiale.

4. Dispositif (20) selon l'une des revendications 1 à 3, **caractérisé en ce que** l'enveloppe externe (22) peut être fixée au moyen d'un dispositif de blocage sur une tige (13) de l'instrument chirurgical (10).

5. Dispositif (20) selon l'une des revendications 1 à 4, **caractérisé en ce que** la partie avant de l'enveloppe externe (22) est fermée.

6. Dispositif 20, selon l'une des revendications 1 à 5, **caractérisé en ce que** l'enveloppe externe (22) est formée de métal noble et/ou de matière synthétique de degrés de dureté différents.

7. Dispositif (20) selon l'une des revendications 1 à 6, **caractérisé en ce que** l'enveloppe externe (22) est symétrique en rotation.

8. Dispositif selon l'une des revendications 1 à 7, **caractérisé en ce que** l'enveloppe externe (22) présente essentiellement une forme conique et/ou d'olive.

9. Dispositif (20) selon l'une des revendications 1 à 8, **caractérisé en ce que** l'enveloppe externe (22) peut être contrôlée au moyen du dispositif de traction (21) par la poignée (15) et/ou le levier (17) de l'instrument chirurgical (10).
